# EUROPÄISCHE PATENTANMELDUNG

(11) **EP 0 694 522 A2**
(43) Veröffentlichungstag der Anmeldung: **31.01.1996**
(21) Anmeldenummer: 95106231.4
(22) Anmeldetag: 26.04.1995
(51) Int. Cl.: C07C 51/10, C07C 57/58, C07C 67/313, C07C 69/614, C07C 69/62

(54) **Verfahren zur Herstellung von Arylessigsäurederivaten durch Carbonylierung von Arylmethylhalogeniden**

(30) Priorität: 04.05.1994 DE 4415681
(71) Anmelder: HOECHST AKTIENGESELLSCHAFT, D-65929 Frankfurt am Main (DE)
(72) Erfinder: Kohlpaintner, Christian Dr., Hoechst Celanese Corp, Corpus Christi TX 78469-9077 (DE); Beller, Matthias, Dr., D-65510 Idstein (DE)

(57) **Zusammenfassung**

Die Erfindung betrifft ein Verfahren zur Herstellung von Arylessigsäurederivaten der allgemeinen Formel (I) 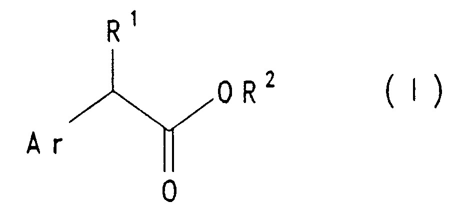
worin
- R¹: Wasserstoff, Halogen, 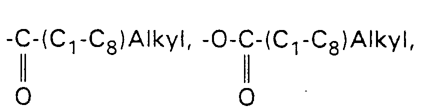 (C₁-C₈)-Alkyl, Phenyl die auch mit Hydroxy- und Aminogruppen substituiert sein können,
- R²: Wasserstoff, (C₁-C₈)-Alkyl, Phenyl,
bedeuten, und
- Ar: für mit Halogen, OH, Polyfluor-(C₁-C₈)-alkyl, CN, (C₁-C₄)-Alkoxy, NO₂, COO-(C₁-C₄)Alkyl, CON[(C₁-C₄)Alkyl]₂, NH(C₁-C₄)Alkyl, N[(C₁-C₄)Alkyl]₂, NH₂, SO₃H, SO₃(C₁-C₄)Alkyl, SO₂NH₂, O-CO-(C₁-C₄)Alkyl, CH₂-Halogen substituiertes oder unsubstituiertes Phenyl, Naphthyl, Anthracenyl, Phenanthrenyl steht, dadurch gekennzeichnet, daß Verbindungen der Formel (II) 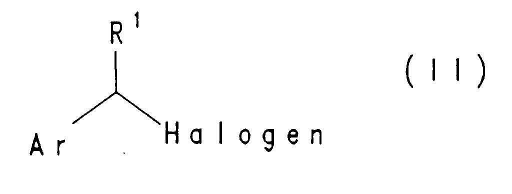
worin Ar und R¹ die oben angeführten Bedeutung besitzen und Halogen für I, Br, Cl steht, mit einem wasserlöslichen Metallkomplex als Katalysator in einem Zweiphasensystem carbonyliert werden.

## Beschreibung

Die Erfindung betrifft ein Verfahren zur Herstellung von Arylessigsäurederivaten durch Carbonylierung von Arylmethylhalogeniden
Arylessigsäuren und ihre Derivate sind wertvolle Zwischenprodukte für die Herstellung von Arzneimitteln, Riech- und Aromastoffen.

Arylessigsäuren können aus den entsprechenden Benzylhalogeniden dargestellt werden, indem diese durch Cl/CN-Austausch in die Benzylcyanide übergeführt und in einer zweiten Verfahrensstufe zu den Arylessigsäuren verseift werden.

Das Verfahren ist beispielsweise in Römpp Chemie Lexikon 9. Auflage 1991, Bd. 4, Seite 3361 bzw. Ullmann 4. Auflage 1976, Bd. 11, Seite 71 beschrieben. Nachteilig sind die Verwendung von NaCN für den Cl/CN-Austausch, der zweimalige Salzanfall (1. Stufe NaCl, 2. Stufe (NH₄)₂SO₄) und die Fahrweise in einem zweistufigen Prozeß.

Vorteilhafter ist die Umsetzung von Benzylhalogeniden mit Kohlenmonoxid, die in einem Einstufenverfahren direkt die Arylessigsäuren zugänglich macht. Diese Reaktion wird durch Metalle der VIII. Nebengruppe katalysiert und ist dadurch gekennzeichnet, daß der Katalysator homogen gelöst, zusammen mit dem Edukt, in einer organischen Phase vorliegt. Die während der Reaktion intermediär gebildete Carbonsäure wird durch die gleichzeitige Anwesenheit einer wäßrigen basischen Lösung in das Salz übergeführt und in dieser wäßrigen Phase auch gelöst. Am Ende der Reaktion wird die wäßrige, das Salz der Carbonsäure enthaltende Phase abgetrennt und die freie Säure durch Ansäuern mittels einer geeigneten Mineralsäure und Extraktion mit einem geeigneten, nicht mit Wasser mischbaren Lösungsmittel gewonnen.

Beispielsweise wird gemäß FR 1 313 360 als Katalysator das Natriumsalz des Tetracarbonylcobaltats [NaCo(CO)₄] zusammen mit einem Amin verwendet. Die IT 14 359 beschreibt die Verfahrensweise mit Ni(CO)₄ in DMF oder DMSO unter Anwesenheit von lodiden und Erdalkalioxiden wie CaO und MgO. Die Verwendung von 5 % Palladium auf Aktivkohle wird in JP 04 661 221 beschrieben. Die Carbonylierungsreaktion kann auch gemäß DE 2 240 398 mit Co₂(CO)₈ und Metallalkoxiden wie NaOMe durchgeführt werden. An die Stelle von Co₂(CO)₈ kann dabei auch Eisenpentacarbonyl treten (DE 2 259 072). Phosphanmodifizierte Palladium-Komplexe [(PR₃)₂PdCl₂] finden gemäß DE 2 526 046, ebenfalls Verwendung. Für die schnellere Reaktion wird ein Phasentransferreagenz, beispielsweise Tetrabutylammoniumiodid, zugesetzt. Rhodiumkatalysatoren auf Basis RhCl₃ unter Anwesenheit von Iodiden wurden für diese Reaktion ebenfalls beschrieben (DE 2 606 655).

Gemeinsam ist allen diesen Verfahren das Vorliegen des Katalysators in der organischen Phase, während sich durch den Zusatz von wäßriger basischer Lösung das Produkt in der Wasserphase befindet. Die freie Säure wird durch Ansäuern der vorher abgetrennten wäßrigen Phase gewonnen.

Diese Verfahren sind durch eine Reihe von Nachteilen gekennzeichnet. Die Base muß in großem Überschuß zugesetzt werden, da das Zielprodukt nur in Form seines Salzes in der wäßrigen Phase verbleibt. Die während der Reaktion gebildete Säure HX neutralisiert sukzessive die Base und würde bei zu geringem Basenzusatz die Phenylessigsäure freisetzen, deren Löslichkeit im organischen Medium höher ist als in der wäßrigen Phase. Eine Produkt-Katalysatortrennung wäre durch einfache Phasentrennung nicht mehr möglich. Aus diesem Grund muß ein großer Basenüberschuß gewählt werden. Die häufig verwendeten Alkalilaugen wie NaOH verschlechtern in großem Überschuß eingesetzt, die Ausbeute an Phenylessigsäure, da das Edukt Benzylhalogenid gerade im stark alkalischen Bereich schnell zum entsprechenden Benzylalkohol hydrolysiert wird.

Zudem führen stark basische Bedingungen zu einer schnelleren Katalysatordeaktivierung.

Die Reaktion in Zweiphasensystemen organisch/wäßrig wird in der Regel durch die Zugabe von Phasentransferreagenzien beschleunigt. Dazu dienen meist quartäre Ammoniumsalze mit amphiphilen Eigenschaften. Für befriedigende Umsetzungsgeschwindigkeiten werden diese Reagenzien für die Carbonylierung von Benzylhalogeniden zusätzlich zugesetzt.

Bei den vorgegebenen Verfahrensweisen ist die Löslichkeit der Metallkatalysatoren in der wäßrigen Phase zu geringen Teilen gegeben. Dadurch ist ein ständiger Metallverlust durch Austrag mit der Produktphase zu beobachten. Dies führt zu einer sukzessiven Verringerung der Aktivität der Katalysatorlösung. Metallspuren im Produkt rufen zudem eine Qualitätsminderung hervor, die nur durch aufwendige Trennverfahren beseitigt werden kann.

In der Regel sind für befriedigende Umsätze pro Zeiteinheit größere Mengen Katalysator notwendig (1 bis 30 Mol-% Metall bezogen auf das Edukt). Dies wirkt sich zusätzlich negativ auf die Kostenbilanz solcher Verfahren aus. Auch die deutliche Reaktivitätsabstufung I > Br > > Cl im Fall der Benzylhalogenide beschränkt in vielen Fällen die Anwendung des Prozesses auf die meist teuren Benzylbromide und -iodide. Die Selektivität bezüglich der Phenylessigsäure kann sich durch die erhöhte Bildung von Phenylacetaldehyd (vgl. JP 60 019 734) deutlich erniedrigen und diese Verfahren kostenungünstig gestalten.

Das während der Reaktion gebildete Salz der Phenylessigsäure muß nach Abtrennung der wäßrigen Phase mit einer Mineralsäure in die freie Carbonsäure überführt werden. Dafür ist neben dem eigentlichen Katalyseprozeß ein zweiter Verfahrensschritt notwendig. Der Anfall von Salz bei diesem Vorgang führt zu einer Verschlechterung der Ökobilanz. Um die Säure vom gebildetem Salz des Neutralisationsprozesses abzutrennen, ist ein weiterer Verfahrensschritt nötig, beispielsweise eine Extraktion mit einem organischen Lösemittel.

Die Carbonylierung mit wasserlöslichen Katalysatoren wurde bisher in drei Arbeiten angeführt. Die Verwendung von Ruthenium-EDTA-Komplexen ist in J. Mol. Catal. 1988, 44, 179 bis 181 beschrieben. Es handelt sich in diesem Fall allerdings nicht um eine Verfahrensweise in einem zweiphasigen System, da als Lösungsmittel ein Wasser/Ethanol-Gemisch Anwendung findet, dessen Mischungsverhältnis (2:8) sehr genau eingehalten werden muß, um ein Ausfallen des Katalysators zu verhindern. Die Anwesenheit des Alkohols führt konsequenterweise zu Selektivitätseinbußen, da der entsprechende Ester der Phenylessigsäure verstärkt gebildet wird.

Mit tppms (Triphenylphosphanmonosulfonat-Natriumsalz) modifizierte Palladium-Komplexe sind in J. Mol. Catal. 1989, 54, 65 bis 71 beschrieben. Diese Katalysatoren besitzen den Nachteil der deutlich geringeren Löslichkeit in Wasser und der damit erhöhten Solubilität in organischen Lösungsmitteln. Dies führt zur unvollständigen Produkt-Katalysator-Trennung, zu Metallaustrag und zur Minderung der Produktqualität. Das Produkt muß zudem am Ende der Reaktion durch Ansäuern mit HCl in eine organische Phase extrahiert werden.

Die Verwendung von Co₂(CO)₆(tppts)₂-Katalysatoren für die Carbonylierung von Phenylethylbromid ist in Applied Catalysis A: General 1993, 102, 53 bis 67 veröffentlicht. Die bereits erwähnten Nachteile von Cobalt-Katalysatoren kommen auch in diesem Fall zum Tragen: Verwendung großer Mengen Katalysator (8 mol-%), niedrige Selektivität für das Monocarbonylierungsprodukt (d.h. hauptsächlich erfolgt Doppelcarbonylierung zur Phenylbrenztraubensäure) und als Eduktbasis das Bromid, welches ungleich teuerer ist als das entsprechende Chlorid. Auf die toxischen und luftempfindlichen Eigenschaften des teuren Co₂(CO)₈ sei in diesem Zusammenhang hingewiesen.

Es bestand somit ein großer Bedarf nach einem Verfahren, das die beschriebenen Nachteile vermeidet und Arylessigsäuren in hoher Ausbeute und Reinheit in technisch einfach durchführbarer Weise zugänglich macht.

Diese Aufgabe wird gelöst durch ein Verfahren zur Herstellung von Arylessigsäurederivaten der allgemeinen Formel (I) 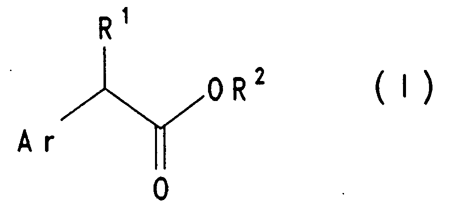
worin
- R¹: Wasserstoff, Halogen, 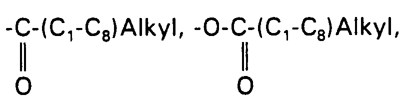 (C₁-C₈)-Alkyl, Phenyl die auch mit Hydroxy- und Aminogruppen substituiert sein können,
- R²: Wasserstoff, (C₁-C₈)-Alkyl, Phenyl,
bedeuten, und
- Ar: für mit Halogen, OH, Polyfluor-(C₁-C₈)-alkyl, CN, (C₁-C₄)-Alkoxy, NO₂, COO-(C₁-C₄)Alkyl, CON[(C₁-C₄)Alkyl]₂, NH(C₁-C₄)Alkyl, N[(C₁-C₄)Alkyl]₂, NH₂, SO₃H, SO₃(C₁-C₄)Alkyl, SO₂NH₂, O-CO-(C₁-C₄)Alkyl, CH₂-Halogen substituiertes oder unsubstituiertes Phenyl, Naphthyl, Anthracenyl, Phenanthrenyl steht, dadurch gekennzeichnet, daß Verbindungen der Formel (II) 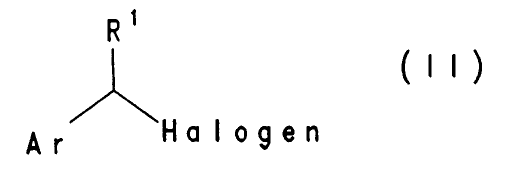
worin Ar und R¹ die oben angeführten Bedeutung besitzen und Halogen für I, Br, Cl steht, mit einem wasserlöslichen Metallkomplex als Katalysator in einem Zweiphasensystem carbonyliert werden.

Als günstig hat sich das Verfahren für Verbindungen erwiesen, worin Ar für 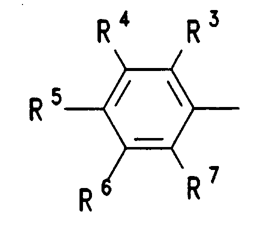
steht, worin R³ bis R⁷ unabhängig voneinander Wasserstoff, Halogen, (C₁-C₄)-Alkyl, OH, CN, Polyfluor-(C₁-C₄)-Alkyl, O(C₁-C₄)Alkyl, NO₂, COO(C₁-C₄)Alkyl, CH₂-Halogen, CON[(C₁-C₄)Alkyl]₂ oder Phenyl bedeuten oder R³ und R⁴, R⁴ und R⁵, R⁵ und R⁶ oder R⁶ und R⁷ zusammen einen aromatischen Ring bilden.

Wichtig sind hierbei die Verbindungen, worin R³ bis R⁷ unabhängig voneinander Wasserstoff, CF₃, Halogen, (C₁-C₄)-Alkyl, (C₁-C₄)-Alkoxy, OH bedeuten.

Als wasserlösliche Metallkomplexe haben sich Verbindungen bewährt, die als Zentralatom ein Metall der VIII. Nebengruppe, insbesondere Palladium, Rhodium, Cobalt, Nickel, Eisen, bevorzugt Palladium und Cobalt enthalten.

Als Liganden enthält der wasserlösliche Metallkomplex vorzugsweise hydrophile Liganden, insbesondere hydrophile Phosphane und Amine, bevorzugt sulfonierte Mono- und Bisphosphane.

Geeignete hydrophile Liganden sind z.B. in Angew. Chem. 105, 1588-1609 beschrieben.
Hierbei liefern insbesondere Phosphane gute Ergebnisse. Hydrophilie wird bei diesen Verbindungen erreicht durch Einführung von Sulfonsäuregruppen, durch hydrophile Gruppen an der Peripherie, durch quaternisierte Aminoalkyl- und Aminoarylsubstituenten, durch Carboxylierung, durch Hydroxyalkyl- oder Polyethersubstituenten und durch Monoquaternisierung von Bisphosphanen. Wichtige Vertreter dieser Gruppen sind z.B. Phosphinobenzoesäuren, Phosphinoalkanole, Phosphinoalkylphosphoniumsalze, Salze der 3,3',3''-Phosphantriylbenzolsulfonsäure (tppts), Salze der 3-Diphenylphosphinobenzolsulfonsäure (tppms), 1,2-Bis-[bis-(sulfonatophenyl)phosphino]ethan, 2,4-Bis-[bis-(sulfonatophenyl)-phosphino]-pentan, 1,2-Bis-[bis-(sulfonatophenyl)-phosphinomethyl]-cyclobutan, 3,4-Dimethyl-2,5,6-tris-(sulfonatophenyl)-phosphanorborna-2,4-dien, Diphenylphosphinoethansulfonat, 2-Diphenylphosphinoethyltrimethylammoniumsalz, 2,4-Bis-[bis-(trimethylamminophenyl)phosphino]pentan, 3-Diphenylphosphinopropionsäure.

Als besonders günstig hat sich eine Verbindung aus Palladium und Triphenylphosphantrisulfonat-Natrium (tppts) erwiesen.

Der Katalysator wird in Mengen von 0,001 bis 5 Mol-%, insbesondere 0,05 bis 1,0 Mol-%, bevorzugt 0,1 bis 0,5 Mol-% bezogen auf eingesetztes Arylmethylhalogenid verwendet.

Der Katalysator wird dabei vorteilhaft in situ aus einem einfach zugänglichen Metallsalz, z.B. eines Metallhalogenids, -sulfats, -oxids, -nitrats, und dem Liganden hergestellt. Hierbei können auch Gemische verschiedener Liganden, z.B. von Mono- und Bisphosphanen, eingesetzt werden. Hierbei hat es sich in vielen Fällen bewährt, einen 1 bis 40-fachen, insbesondere 2 bis 20-fachen, bevorzugt 3 bis 10-fachen molaren Überschuß an Liganden einzusetzen.

Der besondere Vorteil dieser Vorgehensweise liegt neben der einfachen Durchführbarkeit auch darin, daß ein Verzicht auf die teueren, luftempfindlichen und toxischen Metallcarbonyle, wie z.B. Co₂(CO)₈ möglich ist.

Überraschenderweise können Arylmethylchloride ohne Aktivitätsminderung ebenso carbonyliert werden wie die entsprechenden -bromide und -iodide. Die ökonomische Bilanz ist dadurch deutlich verbessert. Nebenreaktionen, welche die Selektivität und Ausbeute mindern, sind nicht zu beobachten.

Die Reaktion wird in einem stark gerührtem System zweier nicht miteinander mischbaren Lösungsmittel (wäßrig/organisch) durchgeführt. Als organische Lösungsmittel haben sich aromatische und aliphatische Kohlenwasserstoffe, insbesondere Toluol, Xylol, Petrolether, Hexan, iso-Hexan oder Heptan bewährt. Das organische Lösungsmittel wird zweckmäßigerweise so gewählt, daß die entstehende Arylessigsäure darin gut löslich ist.

Die anfangs alkalische wäßrige Lösung wird im Verlauf der Reaktion durch die freiwerdende Säure HX neutralisiert und setzt ab einem bestimmten pH-Wert aus dem gebildeten Salz der Carbonsäure die Phenylessigsäure frei. Anschließende Trennung der organischen von der wäßrigen Phase und Kristallisation der Phenylessigsäure liefert das Produkt in hoher Reinheit und Ausbeute. Die wäßrige Lösung, in der sich der Katalysator befindet, kann erneut für die Reaktion eingesetzt werden.

Es hat sich in vielen Fällen bewährt, bei Temperaturen von 20 bis 110°C, insbesondere 50 bis 90°C zu arbeiten.

Je nach Substitutionsmuster der eingesetzten Arylmethylhalogenide hat sich ein CO-Druck von 1 bis 70 bar als geeignet erwiesen.

Neben reinem Kohlenmonoxid kann auch ein CO/H₂-Gemisch beliebiger Zusammensetzung eingesetzt werden.

Zur Synthese bestimmter substituierter Phenylessigsäuren ist es vorteilhaft, die Base während der Reaktion sukzessive zuzudosieren oder die Reaktion in einem bestimmten pH-Bereich pH-kontrolliert durchzuführen.

Außerdem kann der Zusatz von Phasentransferreagenzien wie Tetraalkylammoniumsalzen oder nukleophilen Reagenzien wie lodiden den Umsatz und/oder die Selektivität des Verfahrens verbessern.

Die Vorteile dieser Vorgehensweise gegenüber den klassischen Verfahren sind vielfältiger Natur. Ein Überschuß an Base ist nicht notwendig, da die Reaktion auch im schwach sauren Bereich abläuft und die Freisetzung der Arylessigsäure erwünscht ist. Damit verbunden ist eine geringere Hydrolyse des Edukts zu dem entsprechenden Arylmethylalkohol. Das Verfahren ist besonders ökonomisch, da die während der Reaktion gebildete Mineralsäure HX für die Freisetzung der Carbonsäure direkt benützt wird und nicht nach der Abtrennung des Salzes extern zugespeist werden muß. Der Salzanfall wird dadurch halbiert und führt zu einer Verbesserung der ökologischen Bilanz des Verfahrens.

Durch die Vermeidung eines großen Basenüberschusses wird die Standzeit des Katalysators gegenüber den klassischen Verfahren deutlich erhöht.

Ein weiterer wesentlicher Vorteil des erfindungsgemäßen Verfahrens ist, daß die Produkte wesentlich geringere Mengen Metallsalze im Vergleich zu den Verfahren des Standes der Technik enthalten (Beispiele 8 und 9).

Die folgenden Beispiele sollen das Verfahren erläutern, ohne es darauf zu beschränken.

### Beispiele

### Beispiel 1: Carbonylierung von Benzylchlorid

5,06 g (40 mmol) Benzylchlorid werden in 30 ml Toluol gelöst und mit einer wäßrigen Lösung von 1,14 g (2 mmol) tppts (= Triphenylphosphantrisulfonat-Natriumsalz) und 45 mg (0,2 mmol) Pd(OAc)₂ in 18 ml Wasser vermengt. Das zweiphasige System wird in einen 200 ml Hastelloy-Autoklaven eingefüllt, mit 5,4 ml (60 mmol) einer 32 %igen NaOH-Lösung versetzt und anschließend bei einem CO-Druck von 20 bar auf 70°C erwärmt. Nach einer Reaktionszeit von 20 Stunden wird der Autoklav abgekühlt, die toluolische Phase abgetrennt und bis zur Trockne eingeengt. Es werden 4,79 g eines weißen kristallinen Materials erhalten, welches das Produkt in 93 % Reinheit enthält. Umkristallisation aus iso-Hexan liefert die Phenylessigsäure. Ausbeute: 4,21 g (30,9 mmol, 77 % d. Th.).

### Beispiel 2: Carbonylierung von Benzylchlorid unter Synthesegas-Bedingungen

Die Reaktion wird entsprechend Beispiel 1 durchgeführt, wobei anstelle von CO ein Gemisch von CO/H₂ (1:1) eingesetzt wird. Ausbeute: 4,33 g (31,8 mmol, 79,5 % d. Th.).

### Beispiel 3: Carbonylierung in einem Zwei-Liter Autoklaven

101,27 g (800 mmol) Benzylchlorid und 0,354 g (2 mmol) PdCl₂ werden in 400 ml Toluol gelöst in den Autoklaven eingefüllt. Anschließend werden 11,43 g (20 mmol) tppts gelöst in 350 ml Wasser sowie 129,5 ml (1,4 mol) einer 32 %igen NaOH-Lösung zugegeben. Der Autoklav wird auf 70°C erwärmt und mit CO auf einen Druck von 15 bar gebracht. Nach 6 Stunden ist die Reaktion, erkennbar am Ende der Gasaufnahme, abgeschlossen. Der Autoklav wird auf Raumtemperatur abgekühlt, entspannt und die organische Phase von der wäßrigen Katalysatorphase abgetrennt. Die organische Lösung wird bis zur Trockne eingeengt. Dabei werden 94,11 g eines weißen Produkts erhalten. Ausbeute nach Umkristallisation aus iso-Hexan: 87,52 g (643 mmol, 80,4 % d. Th.).

### Beispiel 4: Carbonylierung von 4-Fluorbenzylchlorid

11,6 g (80 mmol) 4-Fluorbenzylchlorid und 45 mg (0,2 mmol) Pd(OAc)₂ werden in 50 ml Toluol gelöst und mit 2,29 g (4 mmol) tppts gelöst in 25 ml Wasser versetzt. Das zweiphasige Gemisch wird in einen 250 ml Dreihalskolben überführt und mehrmals mit Kohlenmonoxid gespült. Nach 5 Stunden Reaktionszeit (70°C) ist die Gasaufnahme abgeschlossen. Die toluolische Phase wird abgetrennt und bis zur Trockne eingeengt. Es werden 11,54 g Rohprodukt erhalten, die nach Umkristallisation 10,09 g (65,4 mmol, 81,8 % d. Th.) des Reinprodukts liefern.

### Beispiel 5: Carbonylierung von 2-Flourbenzylchlorid, stufenweise Basenzugabe

Eine Lösung von 8,7 g (60 mmol) 2-Fluorbenzylchlorid in 30 ml o-Xylol wird zusammen mit einer Lösung von 1,14 g (2 mmol) tppts in 10 ml Wasser in einen 250 ml Dreihalskolben überführt und nach Zugabe von 5,6 ml einer 32 %igen NaOH-Lösung (60 mmol) und 35 mg (0,2 mmol) PdCl₂ unter einer CO-Atmosphäre auf 70°C erwärmt. Nach 5 Stunden und weiteren 1,5 Stunden Reaktionszeit werden jeweils 20 mmol NaOH zugegeben. Am Ende der Reaktion (8 Stunden) wird die überstehende organische Phase abgetrennt, bis zur Trockne eingeengt und der Rückstand (8,97 g) aus iso-Hexan umkristallisiert. Reinausbeute: 6,69 g (72,3 % d. Th.).

### Beispiel 6: Carbonylierung von 2-Chlorbenzylchlorid

Die Reaktion wird entsprechend Beispiel 1 mit 2-Chlorbenzylchlorid durchgeführt. Anstelle von Pd(OAc)₂ wird PdCl₂ eingesetzt. Reinausbeute: 4,72 g (27,6 mmol, 69,2 % d. Th.).

### Beispiel 7: Carbonylierung von 2-Methylbenzylchlorid

2,81 g (20 mmol) 2-Methylbenzylchlorid und 45 mg (0,2 mmol) Pd(OAc)₂ werden in 20 ml Toluol gelöst, mit 0,46 g (0,8 mmol) tppts gelöst in 10 ml Wasser versetzt und in einen 250 ml Dreihalskolben unter CO-Atmosphäre auf 70°C erwärmt. Hat der Ansatz die Temperatur erreicht, werden über ein Septum 1,8 ml (20 mmol) einer 32 %igen Lösung von NaOH in Wasser zugegeben. Nach 5 Stunden Reaktionszeit erfolgt eine zweite Zugabe von 20 mmol Base. Nachdem die CO-Aufnahme zum Stillstand kommt (8 Stunden), wird die organische Phase abgetrennt, zur Trockne eingeengt und der Rückstand aus iso-Hexan umkristallisiert. Reinausbeute: 2,47 g (16,4 mmol, 82,8 % d. Th.).

### Beispiel 8:

5,8 g (40 mmol) 2-Fluorbenzylchlorid werden in 40 ml o-Xylol gelöst und zusammen mit einer Lösung von 1,14 g (2 mmol) tppts und 35 mg (0,2 mmol) PdCl₂ in 20 ml Wasser in einen 250 ml Dreihalskolben überführt. Anschließend werden 7,5 g (60 mmol) einer 32 %igen NaOH-Lösung zugegeben. Die Reaktionsmischung wird 16 Stunden bei T = 70°C unter einer CO-Atmosphäre (Normaldruck) gerührt. Am Ende der Reaktionszeit wird die organische Phase abgetrennt und bis zur Trockne eingeengt. Ausbeute 5,83 g (94,5 % d. Th.) der 2-Fluorphenylessigsäure. Im Produkt wird durch Atomabsorptionsspektroskopie (AAS) der Palladium-Gehalt bestimmt. Er beträgt < 1 ppm Palladium.

### Beispiel 9: Vergleichsbeispiel nach Stand der Technik

5,8 g (40 mmol) 2-Fluorbenzylchlorid werden zusammen mit 0,53 g (2 mmol) Triphenylphosphan und 35 mg (0,2 mmol) PdCl₂ in 40 ml o-Xylol gelöst. Dazu werden 7,5 g (60 mmol) einer 32 %igen NaOH-Lösung sowie 20 ml Wasser gegeben. Die Reaktionsmischung wird anschließend 16 Stunden bei T = 70°C unter einer CO-Atmosphäre (Normaldruck) gerührt. Am Ende der Reaktionszeit wird die wäßrige Phase abgetrennt (pH = 13,8). Die organische Phase wird zweimal mit je 20 ml einer alkalischen wäßrigen Lösung extrahiert. Die wäßrigen Lösungen werden vereinigt und mit Salzsäure (p.a.) auf pH 1 gestellt. Die wäßrige Phase wird dreimal mit je 50 ml Ether extrahiert, die Ether-Phasen vereinigt, filtriert und bis zur Trockne eingeengt. Ausbeute: 1,05 g (17,0 % d. Th.) der 2-Fluorphenylessigsäure. Im Produkt wird mittels AAS der Palladium-Gehalt bestimmt. Er beträgt 110 ppm Palladium.

## Patentansprüche

1. Verfahren zur Herstellung von Arylessigsäurederivaten der allgemeinen Formel (I) 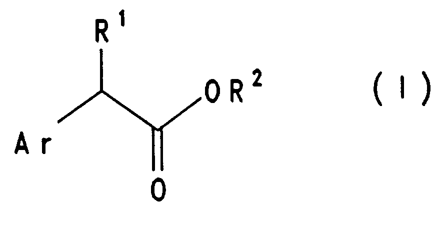 worin
R¹ Wasserstoff, Halogen, 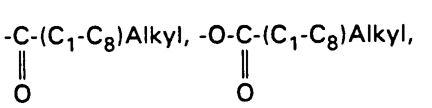 (C₁-C₈)-Alkyl, Phenyl die auch mit Hydroxy- und Aminogruppen substituiert sein können,
R² Wasserstoff, (C₁-C₈)-Alkyl, Phenyl,
bedeuten, und
Ar für mit Halogen, OH, Polyfluor-(C₁-C₈)-alkyl, CN, (C₁-C₄)-Alkoxy, NO₂, COO-(C₁-C₄)Alkyl, CON[(C₁-C₄)Alkyl]₂, NH(C₁-C₄)Alkyl, N[(C₁-C₄)Alkyl]₂, NH₂, SO₃H, SO₃(C₁-C₄)Alkyl, SO₂NH₂, O-CO-(C₁-C₄)Alkyl, CH₂-Halogen substituiertes oder unsubstituiertes Phenyl, Naphthyl, Anthracenyl, Phenanthrenyl steht, dadurch gekennzeichnet, daß Verbindungen der Formel (II) 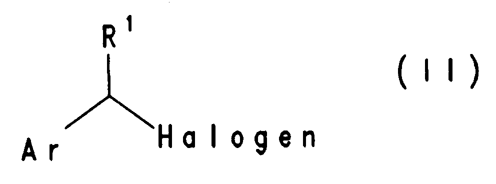
worin Ar und R¹ die oben angeführten Bedeutung besitzen und Halogen für I, Br, Cl steht, mit einem wasserlöslichen Metallkomplex als Katalysator in einem Zweiphasensystem carbonyliert werden.

2. Verfahren nach Anspruch 1, dadurch gekennzeichnet, daß Ar für 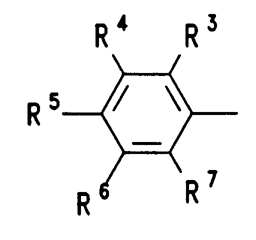 steht, worin R³ bis R⁷ unabhängig voneinander Wasserstoff, Halogen, (C₁-C₄)-Alkyl, OH, CN, Polyfluor-(C₁-C₄)-Alkyl, O(C₁-C₄)Alkyl, NO₂, COO(C₁-C₄)Alkyl, CH₂-Halogen, CON[(C₁-C₄)Alkyl]₂ oder Phenyl bedeuten oder R³ und R⁴, R⁴ und R⁵, R⁵ und R⁶ oder R⁶ und R⁷ zusammen einen aromatischen Ring bilden.

3. Verfahren nach Anspruch 2, dadurch gekennzeichnet, daß R³ bis R⁷ unabhängig voneinander Wasserstoff, CF₃, Halogen, (C₁-C₄)-Alkyl, (C₁-C₄)-Alkoxy, OH bedeuten.

4. Verfahren nach mindestens einem der Ansprüche 1 bis 3, dadurch gekennzeichnet, daß als wasserlösliche Metallkomplexe Verbindungen eingesetzt werden, die als Zentralatom ein Metall der VIII. Nebengruppe, insbesondere Palladium, Rhodium, Cobalt, Nickel, Eisen, bevorzugt Palladium und Cobalt enthalten.

5. Verfahren nach mindestens einem der Ansprüche 1 bis 4, dadurch gekennzeichnet, daß der wasserlöslichen Metallkomplex hydrophile Liganden, enthält.

6. Verfahren nach Anspruch 5, dadurch gekennzeichnet, daß als hydrophile Liganden hydrophile Phosphane und Amine, insbesondere sulfonierte Mono- und Bisphosphane verwendet werden.

7. Verfahren nach Anspruch 5, dadurch gekennzeichnet, daß als Liganden Phosphinobenzoesäuren, Phosphinoalkanole, Phosphinoalkylphosphoniumsalze, Salze der 3,3',3''-Phosphantriylbenzolsulfonsäure (tppts), Salze der 3-Diphenylphosphinobenzolsulfonsäure (tppms), 1,2-Bis-[bis-(sulfonatophenyl)phosphino]ethan, 2,4-Bis-[bis-(sulfonatophenyl)-phosphino]-pentan, 1,2-Bis-[bis-(sulfonatophenyl)-phosphinomethyl]-cyclobutan, 3,4-Dimethyl-2,5,6-tris-(sulfonatophenyl)-phosphanorborna-2,4-dien, Diphenylphosphinoethansulfonat, 2-Diphenylphosphinoethyltrimethylammoniumsalz, 2,4-Bis-[bis-(trimethylamminophenyl)phosphino]pentan, 3-Diphenylphosphinopropionsäure verwendet werden.

8. Verfahren nach mindestens einem der Ansprüche 1 bis 4, dadurch gekennzeichnet, daß als Katalysator eine Verbindung aus Palladium und Triphenylphosphantrisulfonat-Natrium (tppts) eingesetzt wird.

9. Verfahren nach mindestens einem der Ansprüche 1 bis 8, dadurch gekennzeichnet, daß 0,001 bis 5,0 Mol-%, insbesondere 0,05 bis 1,0 Mol-% bevorzugt 0,1 bis 0,5 Mol-% Katalysator bezogen auf eingesetztes Arylmethylhalogenid verwendet werden.

10. Verfahren nach mindestens einem der Ansprüche 1 bis 9, dadurch gekennzeichnet, daß der wasserlösliche Katalysator aus einem Metallsalz und dem Liganden in situ hergestellt wird.

11. Verfahren nach Anspruch 10, dadurch gekennzeichnet, daß bei der in situ Herstellung des Katalysators ein 1 bis 40-facher, insbesondere 2 bis 20-facher, bevorzugt 3 bis 10-facher molarer Überschuß an Liganden eingesetzt wird.

12. Verfahren nach mindestens einem der Ansprüche 1 bis 11, dadurch gekennzeichnet, daß in der allgemeinen Formel (II) Halogen für Chlorid, Bromid oder Jodid, insbesondere Chlorid steht.

13. Verfahren nach mindestens einem der Ansprüche 1 bis 12, dadurch gekennzeichnet, daß die Carbonylierung bei einer Temperatur von 20 bis 110°C, insbesondere 50 bis 90°C durchgeführt wird.

14. Verfahren nach mindestens einem der Ansprüche 1 bis 13, dadurch gekennzeichnet, daß die Carbonylierung bei einem CO-Druck von 1 bis 70 bar durchgeführt wird.

15. Verfahren nach mindestens einem der Ansprüche 1 bis 14, dadurch gekennzeichnet, daß das Zweiphasensystem aus Wasser oder einem Alkohol/Wassergemisch und einem aromatischen oder aliphatischen Kohlenwasserstoff, insbesondere Toluol, Xylol, Hexan, iso-Hexan oder Heptan besteht.
